# EUROPEAN PATENT SPECIFICATION

(11) **EP 4 005 554 B1**
(45) Date of publication and mention of the grant of the patent: **23.09.2026**
(21) Application number: 20763657.2
(22) Date of filing: 19.02.2020
(51) Int. Cl.: A61K 8/9794, C12G 3/022, A61Q 19/10, A61Q 19/00, A61K 8/44

(54) **RICE FERMENTATION LIQUID PRODUCTION METHOD, COSMETIC PRODUCTION METHOD, AND KERATOTIC PLUG REMOVAL METHOD**
VERFAHREN ZUR HERSTELLUNG VON REISFERMENTATIONSFLÜSSIGKEIT, VERFAHREN ZUR HERSTELLUNG VON KOSMETIKA UND VERFAHREN ZUR ENTFERNUNG VON KERATOTISCHEN PFROPFEN
PROCÉDÉ DE PRODUCTION DE LIQUIDE DE FERMENTATION DE RIZ, PROCÉDÉ DE PRODUCTION DE PRODUIT COSMÉTIQUE ET PROCÉDÉ D'ÉLIMINATION DE BOUCHON KÉRATOSIQUE

(30) Priority: 26.02.2019 JP 2019049116
(43) Date of publication of application: 01.06.2022
(73) Proprietor: Minami, Michiko, Fukuoka-shi, Fukuoka 810-0024 (JP)
(72) Inventor: Minami, Michiko, Fukuoka-shi, Fukuoka 810-0024 (JP)
(74) Representative: Valente Cioncoloni, Andrea
(86) International application number: PCT/JP2020/006519
(87) International publication number: WO 2020/175271

(56) References cited:
- EP-A1- 0 514 760
- JP-A- 2000 023 655
- JP-A- 2004 075 575
- JP-A- H02 127 306
- JP-A- H10 339 548
- TANAKA YOSHIHARU ET AL: "Electrolytically generated hydrogen warm water cleanses the keratin-plug-clogged hair-pores and promotes the capillary blood-streams, more markedly than normal warm water does", vol. 8, no. 1, 1 January 2018 (2018-01-01), London, UK, pages 12, XP055977538, ISSN: 2045-9912, Retrieved from the Internet <URL:https://www.medgasres.com/temp/MedGasRes8112-3908541_105125.pdf> DOI: 10.4103/2045-9912.229598
- "NIIGATA, Let's be Echigo Bijin, Visit sake breweries and koji famous spots where you can get cosmetics for beautiful skin", ICOTTO.JP, 20 June 2018 (2018-06-20), XP055735884, Retrieved from the Internet <URL:http://icotto.jp/presses/14909> [retrieved on 20200407]
- "Some cosmetics cannot be handled without a liquor sales license!!", WOMEN THEMSELVES, October 2015 (2015-10-01), XP055735887, Retrieved from the Internet <URL:http://jisin.jp/life/beauty/1619283> [retrieved on 20200407]
- ANONYMOUS: "Not just gourmet! Find "local" cosmetics", 23 June 2016 (2016-06-23), XP009530259, Retrieved from the Internet <URL:https://www.hokende.com/news/blog/entry/exwriter/811> [retrieved on 20200407]
- ITO, YOSHIOM I: "Current Situation and Quality Improvement of YUKIMURO (Snow Storage) Food", KUKI CHOWA EISEI KOGAKU = JOURNAL OF THE SOCIETY OF HEATING, AIR CONDITIONING AND SANITARY ENGINEERS OF JAPAN, vol. 92, no. 5, 2018, JP, pages 45 (409) - 50 (414), XP009530260, ISSN: 0386-4081

## Description

### TECHNICAL FIELD

The present invention relates to a rice fermentation liquid production method comprising a mixing raw material sake and sake less to elute the components of the sake lees into the raw material sake.

### BACKGROUND ART

For some cosmetics such as makeup water and bathing agents, a rice fermentation liquid produced by utilizing the production technique of Japanese Sake is blended as an active ingredient of moisture retention. The rice fermentation liquid abundantly contains amino acids, organic acids, vitamins, and the like, and is said to have moisture retention and whitening effects on the skin.

So far, there is a known technique for obtaining a high amino acid degree sake having a high amino acid degree of 2 or more by mixing sake and sake lees and eluting the component of sake lees into sake and by pressing the sake lees. Such high amino acid degree sake is also used for
cosmetics.

### PRIOR ART LITERATURE

### PATENT DOCUMENT

Patent Document 1: Japanese Patent No. 3635610

### SUMMARY OF THE INVENTION

The invention is set out in the appended set of claims.

### PROBLEM SOLVED BY THE INVENTION

However, there remains room for stably producing a high-quality rice fermentation liquid for a cosmetic.

It is therefore an object of the present invention to provide a rice fermentation liquid production method capable of stably producing a high-quality rice fermentation liquid.

### MEANS FOR SOLVING THE PROBLEMS

The present invention discloses a rice fermentation
liquid production method comprising a mixing step of mixing raw material sake and sake lees to elute the components of the sake lees into the raw material sake, further comprising a low-temperature storage step of storing a high amino acid containing liquid at a low temperature of 4°C or lower. In said low-temperature storage step the liquid in the first container in the second container is stored.

The third aspect of the present invention is rice fermentation liquid production method of the second aspect, wherein, in the housing step, the salt water mixture in a slurry state is used.

The fourth aspect of the present invention is rice fermentation liquid production method of any one of the first through third aspects, wherein, in the low-temperature storage step, liquid is
covered with a special sheet having a heat-insulating function and a light-shielding function.

The fifth aspect of the present invention is rice fermentation liquid production method of any one of the first through fourth aspects, wherein, in the low-temperature storage step, liquid is stored in a snow storage.

The sixth aspect of the present invention is the rice fermentation liquid production method of any one of the first through fifth aspects, wherein, in the low-temperature storage step, liquid is stored at between 1-3°C.

The seventh aspect of the present invention is the rice fermentation liquid production method of any one of the first through sixth aspects, wherein, in the low-temperature storage step, a period for storing
is 3 months or more.

The eighth aspect of the present invention is the rice fermentation liquid production method of any one of the first through seventh aspects wherein the mixing step is repeated a plurality of times before the low-temperature storage step.

The ninth aspect of the present invention is a method of producing a cosmetic containing a rice fermentation liquid, wherein the rice fermentation liquid is produced by rice fermentation liquid production method of any one of the first through eighth aspects.

The tenth aspect of the present invention is a keratotic plug removal method for removing a keratotic plug with a cosmetic containing rice fermentation liquid, wherein the rice fermentation liquid is produced by the rice fermentation liquid production method of any one of the first through eighth aspects, and comprising a first coating step of applying the cosmetic to a target region where the keratotic plug is present and the vicinity thereof.

The eleventh aspect of the present invention is the keratotic plug removal method of the tenth aspect, further comprising, prior to the first coating step, a warming step of warming the target region.

The twelfth aspect of the present invention is the keratotic plug removal method of the tenth or eleventh aspect, further comprising, after the first coating step, a first pressurizing step of pressurizing the target region, a second coating step of applying the cosmetic to the target region, and a second pressurizing step of pressurizing the target region at a pressure higher than that of the first pressurizing step.

The thirteenth aspect of the present invention is the keratotic plug removal method of the twelfth aspect, wherein the second pressurizing step is performed over a period of time more than the second coating step.

### EFFECT OF THE INVENTION

According to each aspect of the present invention, the raw material sake can be stored in a stable environment free from vibration and temperature change, and it becomes possible to stably produce a high-quality rice fermentation the amino acid. It is also possible to effectively remove the keratotic plug by the obtained cosmetic.

According to the second aspect of the present invention, the raw material sake can be stored in a lower temperature state with less temperature change, leading to obtaining the rice fermentation liquid with an active ingredient such as an amino acid at a higher concentration.

According to the fourth aspect of the present invention, for example, it is possible to prevent heat transfer and light incidence from the outside to the high amino acid containing liquid in the first container, even if the user goes into and out of the snow storage in periodic maintenance or the like, and to keep the temperature of the high amino acid containing liquid constant.

### BRIEF DESCRIPTION OF THE DRAWINGS

FIG. 1 is a flow diagram of the rice fermentation liquid and a cosmetic production method according to the first embodiment.
FIG. 2 is a view showing the inside of a snow storage used in the rice fermentation liquid production method of the present invention.
FIG. 3 is a flow diagram of the rice fermentation liquid and a cosmetic production method according to the second embodiment.
FIG. 4 is a schematic diagram showing a storage state of the rice fermentation liquid according to the second embodiment.
FIG. 5 is a diagram showing keratotic plug removed by the keratotic plug removal method of the first embodiment and the second embodiment.

### FORM FOR CARRYING OF THE INVENTION

An embodiment of the present invention will be described in detail below. The Sake (hereinafter referred to as "raw material sake") used as a raw material in the present invention is described. The sake used in the present invention is not limited to pure rice sake. The type of the raw material sake is not particularly limited and may be ginjoshu, honjozoshu, ordinary sake, zojoshu or the like.

### Embodiment 1

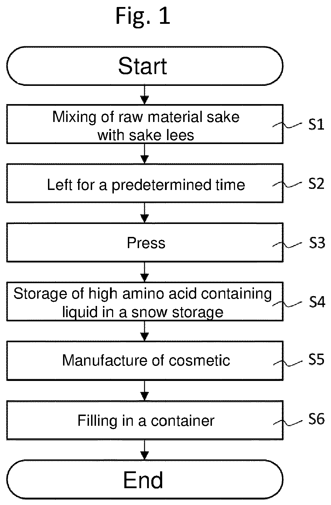

FIG. 1 is a flow diagram of the rice fermentation liquid and a cosmetic production method according to the present invention. Steps S1 to S3 for obtaining high amino acid containing liquid are referred to Patent Document 1.

First, Sake lees and the raw Sake are mixed (step S1; An example of an "mixing step" in CLAIMS.) In order to effectively elute the components of the sake lees, especially the amino acids, into the raw sake, it is favorable to add the raw material sake with the sake lees in a kneading machine little by little to prepare the kneaded lees of the raw material sake. Then, the kneaded lees of the raw material sake is placed in a tank in which the raw material sake is stored and stirred until it becomes uniform using a submerged pump. Since the amino acid degree increases in proportion to the sake lees concentration, the concentration of sake lees may be adjusted according to the desired amino acid degree.

The raw material liquid in which sake lees has been uniformly mixed is placed for a desired time, for example, overnight (step S2). The leaving atmosphere may be at room temperature (5-10°C.) in the sake storagewhich the components of the sake lees are eluted (step S3). The sake lees residue is effectively removed by pressing in the same manner as in the sake manufacturing process. The liquid obtained by the pressing (hereinafter referred to as "high amino acid containing liquid") is transparent and a particularly high amino acid degree is obtained.

In order to produce high amino acid containing liquid having a even higher amino acid degree, the process of steps S1-S3 is more effective when repeatedly executed for several times than mixing a large amount of sake lees at one time into the raw material sake. For example, when sake lees of 200kg should be eluted, high amino acid containing liquid with higher concentration can be obtained if divided into plurality of times such as 50kg, 50kg and 100kg and mixed step by step, than being eluted at one time.

The high-amino-acid sake obtained as described above is heated to 60-65°C in the same manner as the conventional sake manufacturing method if the raw material sake is not heated, thereby promoting the death of the yeast and deactivation of the enzyme to maintain the quality.

Subsequently, as soon as the high amino acid containing liquid is prepared, the high amino acid containing liquid is covered with a special sheet having heat insulating function and light shielding function, and is stored immediately in a snow storage (step S4; An example of a "low-temperature storage step" in CLAIMS). When a rice fermentation liquid (An example of a "rice fermentation liquid" in CLAIMS) completed through a storage period for about three months is mixed with other cosmetic raw materials (Step S5) and filled in a container, the cosmetic (An example of a "cosmetic" in CLAIMS) is completed.

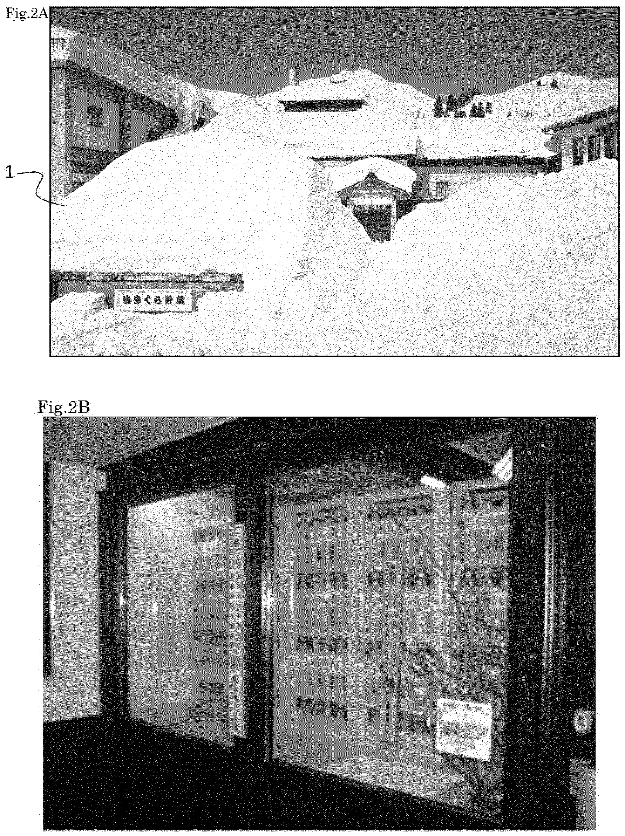

FIG. 2 is a view showing (A) the outside and (B) the inside of the snow storage 1 used in the rice fermentation liquid production method of the present invention. The snow storage 1 is kept at a temperature of 4°C or less, specifically 1 to 3 degrees, more preferably about 2°C. There is little in temperature change and little vibration in the snow storage 1, which enables stable quality control.

Table 1 is a table showing the amino acid content of the rice fermentation liquid produced by the rice fermentation liquid production method of the present invention. The rice fermentation liquid according to the present invention contains an amino acid having a concentration of about 5 times as compared with sake according to a conventional production method. Since about 70% of the total amino acid content is a natural moisturizing factor (moisturizing amino acid), the rice fermentation liquid according to the present invention has an excellent moisturizing effect.

**[Table 1]**

| Ingredient name | Content |
|---|---|
| Free amino acid | |
| Free Arginine | 42mg/100g |
| Free Lysine | 38mg/100g |
| Free Histidine | 12mg/100g |
| Free Phenylalanine | 35mg/100g |
| Free Tyrosine | 37mg/100g |
| Free Leucine | 61mg/100g |
| Free Isoleucine | 32mg/100g |
| Free Methionine | Not detected |
| Free Valine | 48mg/100g |
| Free Alanine | 84mg/100g |
| Free Glycine | 38mg/100g |
| Free Proline | 33mg/100g |
| Free Glutamic acid | 31mg/100g |
| Free Serine | 37mg/100g |
| Free Threonine | 27mg/100g |
| Free Aspartic acid | 57mg/100g |

The cosmetic produced by the cosmetic production method according to the present invention is also useful for removing waste or unnecessary substances at least a part of which is embedded in the skin surface of an animal. Here, as a representative example of such waste or unnecessary materials, a keratotic plug is referred to. In particular, a first coating step (An example of a "first coating step" in CLAIMS) after a warming step (An example of a "warming step" in CLAIMS) of warming a target region where the keratotic plug is present and the vicinity thereof is effective. The first coating step is the step of applying the cosmetic so as to be spread around the target region with the fingertip moving in circles around. As a result, the keratotic plug can be easily removed.

Further, it is even easier to remove the keratotic plug through a first pressurizing step (An example of a "first pressurizing step" in CLAIMS) of rubbing the target region while pressurizing the target region, a step of applying the above cosmetic to the target region for two minutes or more (An example of the "second coating step" in CLAIMS), and a second pressurizing step of rubbing the target region while applying a pressure higher than the first pressure step.

It is favorable to use the raw material sake, as the raw material liquid, produced before heating in a conventional method, but the liquid produced after heating may be used. In addition, sake lees are not particularly limited, either. Ordinary sake lees can be used, and it is favorable to use fresh sake lees.

In the step S1, sake lees may be directly put in a predetermined amount of raw material liquid and stirred and mixed.

Further, in the step S2, the leaving time depends on the elution rate of the sake lees component, but is not particularly limited. For example, the material liquid may be left for 2 hours or 4 days.

Further, since the amino acid is also a raw material of deliciousness, the rice fermentation liquid of the present invention may be used as a seasoning as it is.

In addition, the cosmetic may be not only makeup water or a bathing agent but also milky lotion, cream, cosmetic liquid, makeup cosmetic, a soap, a cleansing agent or the like.

### Embodiment 2

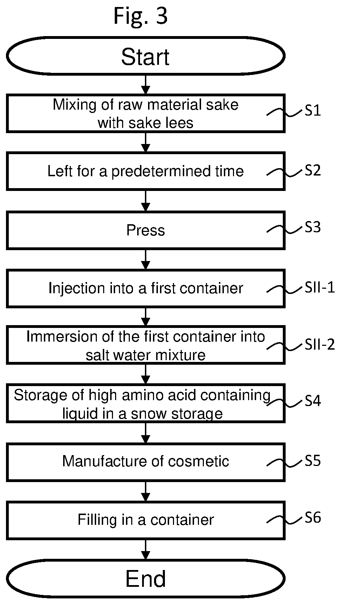

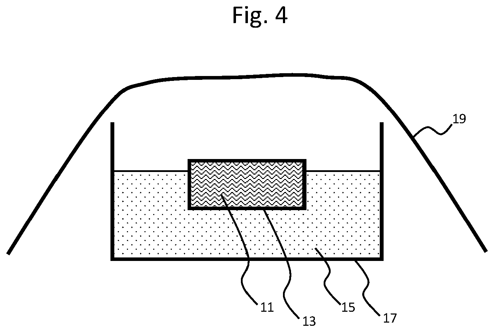

FIG. 3 is a flow diagram of the rice fermentation liquid and the cosmetic production method according to the second embodiment. FIG. 4 is a schematic diagram showing a storage state of the rice fermentation liquid. First, as shown in FIG. 4, prior to step S4 in Embodiment 1, the high amino acid containing liquid 11 obtained through step S3 is injected into the first container 13 (step SII-1; An example of a "first injection step" in CLAIMS). And the first container 13 is immersed in the second container 17 containing a salt water mixture 15 containing salt and water (step SII-2; An example of a "housing step" in CLAIMS). In step S4, the second container 17 is covered with a special sheet 19 having a heat insulating function and a light shielding function. Then, the second container 17 is stored in the snow storage. Other steps are the same as in Embodiment 1. For the special sheet, a sheet coated with aluminum or aluminum foil can be used. As a result, it is possible to block the influence of light and heat to the first container even when users come in or out the low-temperature storage such as a snow storage for periodic maintenance or the like.

For the salt water mixture, a salt water mixture with a salt concentration of 35-40% in a slurry state was used.

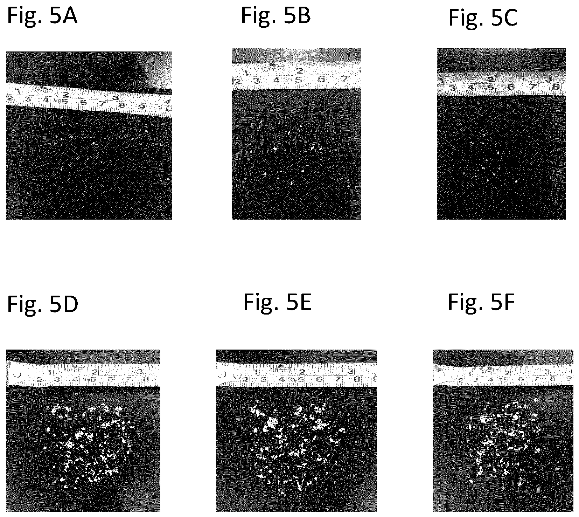

The keratotic plug is removed with the cosmetic of Embodiment 1 and Embodiment 2, and the numbers of keratotic plugs removed from the region of the cheek 8 cm × 8 cm of the subject were counted visually. While it was impossible to remove keratotic plugs using the cosmetic containing conventional rice fermentation liquid, 10 to 15 keratotic plugs were removed when using the cosmetic of Embodiment 1. As many as 100 to 126 keratotic plugs were removed when using the cosmetic of Embodiment 2. FIG. 5 shows three examples of square plugs removed by the keratotic plug removal method according to Embodiment 1 and Embodiment 2. FIG. 5A-5C show the keratotic plugs removed by the keratotic plug removal method of Embodiment 1. FIG. 5D-5F show keratotic plugs removed by the keratotic plug removal method of Embodiment

2. As clearly shown in FIG. 5, it can be seen that the
keratotic plug removal method of Embodiment 2 is able to remove a much greater number of angular plugs than the keratotic plug removal method of Embodiment 1.

As described above, by storing the high amino acid
containing liquid in the container immersed in the salt water mixture, it is possible to maintain a lower temperature change at a lower temperature than in Embodiment 1. This results in an increase in the amount of amino acids, and an advantageous effect in the removal of keratotic plugs.

The first container and the second container are not limited to a box shape as shown in FIG. 3 (b). They may be of a bag shape made of a thin film.

1 Snow Storage, 11 High Amino Acid Containing Liquid, 13 First Container, 15 Salt Water Mixture, 17 Second Container, 19 Special Sheet.

## Claims

1. A rice fermentation liquid production method comprising a mixing step of mixing raw material sake and sake lees to elute the components of the sake lees into the raw material sake, further comprising:
a first injection step of injecting liquid containing a high amount of amino acid obtained by the mixing step into a first container;
a housing step of accommodating the first container in a second container containing a salt water mixture containing salt and water; and
a low-temperature storage step of storing the liquid at a low temperature of 4°C or lower, and
wherein, in the low-temperature storage step, the liquid in the first container in the second container is stored.

2. The rice fermentation liquid production method of claim 1, wherein, in the housing step, the salt water mixture in a slurry state is used.

3. The rice fermentation liquid production method of claim 1 or 2, wherein, in the low-temperature storage step, the liquid is covered with a special sheet having a heat-insulating function and a light-shielding function.

4. The rice fermentation liquid production method of any one of claim 1 through 3, wherein, in the low-temperature storage step, the liquid is stored in a snow storage.

5. The rice fermentation liquid production method of any one of claim 1 through 4, wherein, in the low-temperature storage step, the liquid is stored at between 1-3°C.

6. The rice fermentation liquid production method of any one of claim 1 through 5, wherein, in the low-temperature storage step, a period for storing the liquid is 3 months or more.

7. The rice fermentation liquid production method of any one of claim 1 through 6, wherein the mixing step is repeated a plurality of times before the low-temperature storage step.

8. A method of producing a cosmetic containing a rice fermentation liquid, wherein the rice fermentation liquid is produced by the rice fermentation liquid production method of any one of claims 1 through 7.

9. A keratotic plug removal method for removing a keratotic plug with a cosmetic containing rice fermentation liquid,
wherein the rice fermentation liquid is produced by the rice fermentation liquid production method of any one of claims 1 through 7, and comprising:
a first coating step of applying the cosmetic to an target region where the keratotic plug is present and the vicinity thereof.

10. The keratotic plug removal method of claim 9, further comprising, prior to the first coating step, a warming step of warming the target region.

11. The keratotic plug removal method of claim 9 or 10, further comprising, after the first coating step:
a first pressurizing step of pressurizing the target region;
a second coating step of applying the cosmetic to the target region; and
a second pressurizing step of pressurizing the target region at a pressure higher than that of the first pressurizing step.

12. The keratotic plug removal method of claim 11, wherein the second pressurizing step is performed over a period of time more than the second coating step.

## Patentansprüche

1. Verfahren zur Herstellung einer Reisfermentationsflüssigkeit, umfassend einen Mischschritt des Mischens von Ausgangsmaterial-Sake und Sake-Trester, um die Komponenten des Sake-Tresters in den Ausgangsmaterial-Sake zu eluieren, ferner umfassend:
einen ersten Injektionsschritt des Injizierens von Flüssigkeit, die eine durch den Mischschritt erhaltene hohe Menge an Aminosäure enthält, in einen ersten Behälter;
einen Unterbringungsschritt des Unterbringens des ersten Behälters in einem zweiten Behälter, der eine Salzwassermischung enthält, die Salz und Wasser enthält; und
einen Niedertemperatur-Lagerungsschritt des Lagerns der Flüssigkeit bei einer niedrigen Temperatur von 4 °C oder weniger, und
wobei im Niedertemperatur-Lagerungsschritt die Flüssigkeit in dem ersten Behälter in dem zweiten Behälter gelagert wird.

2. Verfahren zur Herstellung einer Reisfermentationsflüssigkeit nach Anspruch 1, wobei im Unterbringungsschritt die Salzwassermischung in einem Aufschlämmungszustand verwendet wird.

3. Verfahren zur Herstellung einer Reisfermentationsflüssigkeit nach Anspruch 1 oder 2, wobei im Niedertemperatur-Lagerungsschritt die Flüssigkeit mit einer Spezialfolie abgedeckt wird, die eine wärmeisolierende Funktion und eine lichtabschirmende Funktion aufweist.

4. Verfahren zur Herstellung einer Reisfermentationsflüssigkeit nach einem der Ansprüche 1 bis 3, wobei im Niedertemperatur-Lagerungsschritt die Flüssigkeit in einem Schneelager gelagert wird.

5. Verfahren zur Herstellung einer Reisfermentationsflüssigkeit nach einem der Ansprüche 1 bis 4, wobei im Niedertemperatur-Lagerungsschritt die Flüssigkeit bei zwischen 1 und 3 °C gelagert wird.

6. Verfahren zur Herstellung einer Reisfermentationsflüssigkeit nach einem der Ansprüche 1 bis 5, wobei im Niedertemperatur-Lagerungsschritt ein Zeitraum zur Lagerung der Flüssigkeit 3 Monate oder mehr beträgt.

7. Verfahren zur Herstellung einer Reisfermentationsflüssigkeit nach einem der Ansprüche 1 bis 6, wobei der Mischschritt vor dem Niedertemperatur-Lagerungsschritt mehrmals wiederholt wird.

8. Verfahren zur Herstellung eines Kosmetikums, das eine Reisfermentationsflüssigkeit enthält, wobei die Reisfermentationsflüssigkeit durch das Verfahren zur Herstellung einer Reisfermentationsflüssigkeit nach einem der Ansprüche 1 bis 7 hergestellt wird.

9. Verfahren zur Entfernung von Hornpfröpfen zum Entfernen eines Hornpfropfens mit einem Kosmetikum, das Reisfermentationsflüssigkeit enthält,
wobei die Reisfermentationsflüssigkeit durch das Verfahren zur Herstellung einer Reisfermentationsflüssigkeit nach einem der Ansprüche 1 bis 7 hergestellt wird, und umfassend:
einen ersten Beschichtungsschritt des Auftragens des Kosmetikums auf eine Zielregion, in der der Hornpfropfen vorhanden ist, und deren Umgebung.

10. Verfahren zur Entfernung von Hornpfröpfen nach Anspruch 9, ferner umfassend, vor dem ersten Beschichtungsschritt, einen Erwärmungsschritt des Erwärmens der Zielregion.

11. Verfahren zur Entfernung von Hornpfröpfen nach Anspruch 9 oder 10, ferner umfassend, nach dem ersten Beschichtungsschritt:
einen ersten Druckbeaufschlagungsschritt des Beaufschlagens der Zielregion mit Druck;
einen zweiten Beschichtungsschritt des Auftragens des Kosmetikums auf die Zielregion; und
einen zweiten Druckbeaufschlagungsschritt des Beaufschlagens der Zielregion mit einem Druck, der höher ist als der des ersten Druckbeaufschlagungsschritts.

12. Verfahren zur Entfernung von Hornpfröpfen nach Anspruch 11, wobei der zweite Druckbeaufschlagungsschritt über einen längeren Zeitraum als der zweite Beschichtungsschritt durchgeführt wird.

## Revendications

1. Procédé de production de liquide de fermentation de riz comprenant une étape de mélange du saké brut et des lies de saké afin d'éluer les composants des lies dans le saké brut, incluant en outre :
une première étape d'injection du liquide riche en acides aminés obtenu lors de l'étape de mélange dans un premier récipient ;
une étape de conservation consistant à placer le premier récipient dans un second récipient qui contient une solution saline ;
une étape de stockage à basse température consistant à conserver le liquide à une température inférieure ou égale à 4 °C ;
au cours de cette étape de stockage à basse température, le liquide du premier récipient est conservé dans le second récipient.

2. Procédé de production de liquide de fermentation de riz selon la déclaration 1, **caractérisé en ce que**, lors de l'étape de stockage, un mélange d'eau salée à l'état de suspension est utilisé.

3. Procédé de production de liquide de fermentation de riz selon la déclaration 1 ou 2, **caractérisé en ce que**, lors de l'étape de stockage à basse température, le liquide est recouvert d'une feuille spéciale présentant une fonction d'isolation thermique et d'opacité.

4. Procédé de production de liquide de fermentation de riz selon l'une quelconque des déclarations 1 à 3, **caractérisé en ce que**, lors de l'étape de stockage à basse température, le liquide est stocké dans un récipient rempli de neige.

5. Procédé de production de liquide de fermentation de riz selon l'une quelconque des déclarations 1 à 4, **caractérisé en ce que**, lors de l'étape de stockage à basse température, le liquide est stocké entre 1 et 3 °C.

6. Procédé de production de liquide de fermentation de riz selon l'une quelconque des déclarations 1 à 5, **caractérisé en ce que**, lors de l'étape de stockage à basse température, la durée de stockage du liquide est de 3 mois ou plus.

7. Procédé de production de liquide de fermentation de riz selon l'une quelconque des déclarations 1 à 6, **caractérisé en ce que** l'étape de mélange est répétée plusieurs fois avant l'étape de stockage à basse température.

8. Procédé de fabrication d'un cosmétique contenant un liquide de fermentation de riz, ce liquide étant produit selon le procédé de production de liquide de fermentation de riz selon l'une quelconque des déclarations 1 à 7.

9. Procédé d'élimination d'un bouchon kératosique à l'aide d'un cosmétique contenant un liquide de fermentation de riz,
ce liquide étant produit selon le procédé de production de liquide de fermentation de riz selon l'une quelconque des déclarations 1 à 7, et incluant :
une première étape d'application du cosmétique sur la zone cible où se trouve le bouchon kératosique et ses alentours.

10. Procédé d'élimination d'un bouchon kératosique selon la déclaration 9, comprenant en outre, avant la première étape d'application, une étape de réchauffement de la zone cible.

11. Procédé d'élimination du bouchon kératosique selon la déclaration 9 ou 10, comprenant en outre, après la première étape d'application :
une première étape de pressurisation de la zone cible ;
une deuxième étape d'application consistant à poser le produit cosmétique sur la zone cible ;
une deuxième étape de pressurisation de la zone cible à une pression supérieure à celle de la première étape de pressurisation.

12. Procédé d'élimination du bouchon kératosique selon la déclaration 11, dans lequel la deuxième étape de pressurisation est effectuée sur une période de temps supérieure à celle de la deuxième étape d'application.
